Europäisches Patentamt

European Patent Office

Office européen des brevets

(11) Publication number: **0 280 728 B1**

# EUROPEAN PATENT SPECIFICATION

(45) Date of publication of patent specification: **20.01.93**   (51) Int. Cl.5: **A61M 29/00**

(21) Application number: **87906949.0**

(22) Date of filing: **06.08.87**

(86) International application number:
**PCT/US87/01957**

(87) International publication number:
**WO 88/00844 (11.02.88 88/04)**

(54) **ANGIOPLASTY DILATING GUIDE WIRE.**

(30) Priority: **08.08.86 US 894658**

(43) Date of publication of application:
**07.09.88 Bulletin  88/36**

(45) Publication of the grant of the patent:
**20.01.93 Bulletin  93/03**

(84) Designated Contracting States:
**AT BE CH DE FR GB IT LI NL SE**

(56) References cited:
| | |
|---|---|
| WO-A-86/06285 | US-A- 4 276 874 |
| US-A- 4 299 226 | US-A- 4 315 512 |
| US-A- 4 423 725 | US-A- 4 444 188 |
| US-A- 4 445 892 | US-A- 4 453 545 |
| US-A- 4 509 945 | US-A- 4 552 127 |
| US-A- 4 569 332 | US-A- 4 572 186 |
| US-A- 4 573 470 | US-A- 4 582 181 |
| US-A- 4 616 653 | US-A- 4 638 805 |
| US-A- 4 655 746 | |

(73) Proprietor: **SCIMED LIFE SYSTEMS, INC.**
**13000 County Road 6**
**Minneapolis, MN 55441(US)**

(72) Inventor: **WILLARD, Lloyd, K.**
**2851 Pheasant Circle**
**Mound, MN 55364(US)**
Inventor: **EUTENEUER, Charles, L.**
**220 Maple Lane**
**St. Michael, MN 55376(US)**
Inventor: **Kagan, Jonathan**
**5112 Russell Avenue South**
**Minneapolis, Minnesota 55410(US)**

(74) Representative: **Rostovanyi, Peter et al**
**AWAPATENT AB Box 5117**
**S-200 71 Malmö(SE)**

## Description

Field of the Invention

This invention pertains to an angioplasty apparatus according to the first part of claim 1. Such a device is disclosed by the US-A-4 582 181.

Background of the Invention

Angioplasty has gained wide acceptance in recent years as an efficient and effective method for treating certain types of vascular diseases. In particular, angioplasty is widely used for opening of stenoses in the coronary arteries, although it is also used for treatment of stenoses in other parts of the vascular system. Treatment and opening of the stenosis is effected by maneuvering a catheter having an inflatable balloon area near its tip through the vascular system to position the inflatable balloon at the site of the stenosis. The balloon is then inflated to cause stretching of the artery and pressing of the lesion into the artery wall to reestablish acceptable blood flow to the artery.

Guide wires are often used for establishing the path to the stenosis so that the dilating catheter can subsequently be positioned. In percutaneous transluminal coronary angioplasty (PTCA), a steerable guide wire and PTCA catheter may be introduced into the vascular system at a site remote from the stenosis through a guiding catheter and delivered to either the left or right coronary ostia (entrance to the left and right coronary artery) by that guiding catheter. The guide wire is then maneuvered into the branch of the coronary artery for which treatment is intended. After the guide wire has been advanced past the lesion to be treated, the PTCA catheter is then positioned with its hollow central lumen at the distal tip over the end of the guide wire which is outside the body. With the guide wire held in place, the dilating catheter is then advanced over the guide wire to bring the inflatable balloon portion to the lesion. Thereafter, fluid pressure is applied through external apparatus connected to the catheter to inflate the balloon.

Various problems may be encountered in the above-described angioplasty procedure. One problem which occurs in the case of very tight stenoses with small openings is the inability to advance the dilating catheter across the stenosis after the guide wire has been successfully positioned. Since the tip of the dilating catheter has a significantly greater diameter than the guide wire, there is a practical limit to treatment of such small, tight stenoses using transluminal catheters. Efforts to reduce the diameter of the transluminal catheter are limited by the size of the guide wire. Since a clearance is required to allow the guide wire to pass with relatively minimal frictional resistance, one lumen of the transluminal design must have a diameter of at least 0.005 cm (.002 in.) greater than the guide wire to be functional. Guide wires are available in sizes ranging from 0.030 cm to 0.046 cm (.012 in. to .018 in.) in diameter. Reduced diameter guide wires have less axial strength, which may make them subject to buckling as they are being advanced, and reduced torsional stiffness, which makes them more difficult to steer and control during insertion. In addition, very small diameter guide wires might under some circumstances create a risk of injury to the blood vessels because the applied axial force is concentrated over a small tip area, which could create a risk of puncturing the artery and creating false lumens. The standard PTCA catheter, which is passed over the guide wire, is limited in size by the need to allow adequate diametral clearance between the guide wire and the inner diameter of the PTCA through channel.

Another device used in coronary angioplasty is a catheter in which a balloon is installed on a wire with a spring tip. This type of catheter offers slightly lower proflies than a transluminal design, but unlike the transluminal design cannot be used in conjunction with a conventional guide wire. Like a standard guide wire, the tip of these catheters are shaped in order to try to navigate the path from the right or left ostia to and past the vessel to be treated. The primary difficulty associated with this type of device is the inability to duplicate the performances of conventional guide wires, particularly the torsional and axial stiffness, as well as the tip flexibility and smoothness of wire transition from the core to the tip safety wire (which secures the spring tip). The second drawback of this type of device is the fact that when catheter balloon size changes are required because of the extreme difficulty of accurately estimating the artery and occlusion sizes, the entire coronary arterial path must be renegotiated with the next catheter since no guide wire is in place reserving an easily renegotiated path. Once the path has been established, it is highly desirable that the path be maintained, yet the use of single lumen systems requires loss and reestablishment of the path.

U.S. Patent 4,315,512 to Fogarty shows a catheter of this latter type wherein a catheter fits about a guide wire such that a balloon is installed between the end of the guide wire and the end of the catheter. By extending the guide wire further beyond the end of the catheter, the balloon is stretched to give it a low profile. When the balloon is property located, fluid is injected through the annular space between the guide wire and the catheter. If balloon size must be changed, as indicated previously with this type of device, the entire assembly must be removed resulting in a loss of the established path.

The present invention is directed to angioplasty apparatus which allows for the use of multiple balloons without loss of path. Although U.S. Patent 4,655,746 to Daniels et. al. is directed to a different medical purpose than the present invention, it shows a catheter device having a pair of balloons.

Of more interest is U.S. Patent 4,582,181 to Samson. The Samson device has a catheter with a guide wire extending therethrough. A balloon is formed at the distal end of the outer catheter as a part of it and is sealed to the tip portion of the guide wire, the tip extending a short distance beyond the distal end of the balloon. Fluid to inflate the balloon flows in the space between the catheter and the guide wire. Air within the balloon is vented through the hollow guide wire or through a separate tube within the through lumen of the catheter. Although Samson discusses a lower profile than known dilating catheters, the device disclosed has the same problem as the others in that if dilation achieved is insufficient, the entire device must be removed resulting in loss of path.

Thus, of these other problems as outlined above, there is still a need in the field of angioplasty for effective treatment of very tight stenoses with very small openings which cannot be crossed by present angioplasty catheters. Of course, it is highly desirable to be able to treat the small stenosis through percutaneous coronary angioplasty techniques, since the alternative is surgical bypass grafting techniques.

Summary of the Invention

The present invention provides a dilating guide wire that overcomes the problems encountered in the catheters described above. The solution to the problem is stated in the second part of claim 1. The dilating guide wire of the present invention is adapted for use in conjunction with a conventional guide wire and dilating catheter for dilatation of very tight stenoses which can then be subsequently treated with the conventional angioplasty catheter. Use of the present invention does not require loss and reestablishment of the path to the stenosis site. This is accomplished by passing the dilating guide wire through the conventional catheter while it is held in place with its tip immediately proximal the site of the stenosis.

The dilating guide wire of the present invention is configured with an extremely low profile, i.e., a very small diameter, so that it can successfully be advanced and withdrawn through the central lumen of a coaxial conventional angioplasty catheter. Special means are provided for axially extending the balloon segment of the dilation guide wire to flatten the balloon to reduce its profile. This is especially important in allowing the tip to be drawn back into the tip of the conventional dilating catheter at the end of the dilatation, and avoids bunching-up of the dilating balloon which might otherwise occur and prevent withdrawal.

According to another feature of the invention, special means are provided in the form of a hollow core for removal of air from the dilating balloon during preparation with radiopaque liquid and which self-seals during pressurization with the liquid during balloon inflation.

According to another feature of the invention, axial stretching of the tip provides a deflection thereof which can be used for steering during positioning of the dilating tip.

According to another feature of the invention, the central hollow core of the catheter is provided in a stepped configuration with the distal tip section which is extended into the coronary arteries from the PTCA having a smaller outer diameter and cross-sectional area than the main body of the core which is contained within the PTCA. This feature provides an extremely flexible member which is relatively non-traumatic to the coronary artery. This feature also contributes to the next features of this invention.

According to another feature of this invention, the inner diameter of the catheter inflation lumen is stepped to provide optimum hydraulic clearance which is intended for the purpose of providing an acceptable deflation time (time required to remove the entire volume of radiopaque fluid from the balloon after dilatation has been completed, thus restoring blood flow through the dilated channel) after the application of an 80% or greater vacuum. The stepped features of both the central core and the catheter tubings combined provide both the hydraulics and the profile requirement needed to provide a central balloon profile of approximately 0.051cm (.020 inches).

According to another feature of the invention, the inner diameter of the core provides an origination point and structural support for the spring tip safety system.

Brief Description of the Drawing

In the drawings,
FIGURE 1 is a view of a dilating guide wire according to the present invention including its control manifold assembly, portions thereof broken away for purposes of this illustration;

FIGURE 2 is a schematic cross-sectional representation, at an enlarged scale, of a portion of the dilating guide wire of Figure 1 illustrating certain features thereof;

FIGURE 3 is a partially cut-away view at an enlarged scale of the tip portion of the dilating guide wire of Figure 1;

FIGURES 3A and 3B are sections taken as indicated along the tip portion of Figure 3;

FIGURE 4 is a fragmentary view in perspective of a portion of the control manifold assembly of Figure 1;

FIGURE 5 is a sectional view taken along the center axis of the apparatus of Figure 3 with the control knob removed;

FIGURE 6 is a view of a dilating guide wire according to the present invention extending through a PTCA catheter with which it is advantageously used, including control manifolds for both the dilating guide wire and the PTCA;

FIGURE 7 is a schematic representation of the use of the dilating guide wire according to the present invention in conjunction with a PTCA catheter for the treatment of a stenotic lesion in an artery;

FIGURES 8A and 8B are schematic representations in sectional view of the dilating guide wire inserted in the through lumen of the PTCA catheter;

FIGURE 9 is a schematic representation at an enlarged scale of the tip area of a PTCA catheter with the dilating guide wire inserted in the through lumen thereof;

FIGURE 10 is a schematic representation at an enlarged scale of the tip area of a PTCA catheter illustrating attempted withdrawal of the dilating guide wire without the use Of the axial stretch feature of the invention;

FIGURE 11 is a view similar to Figure 10 but with the use of the axial stretch feature of the invention; and

FIGURE 12 is a view of the tip portion of the dilating guide wire illustrating the controlled deflection thereof through the use of the axial stretch feature of the invention.

## Detailed Description of the Preferred Embodiment

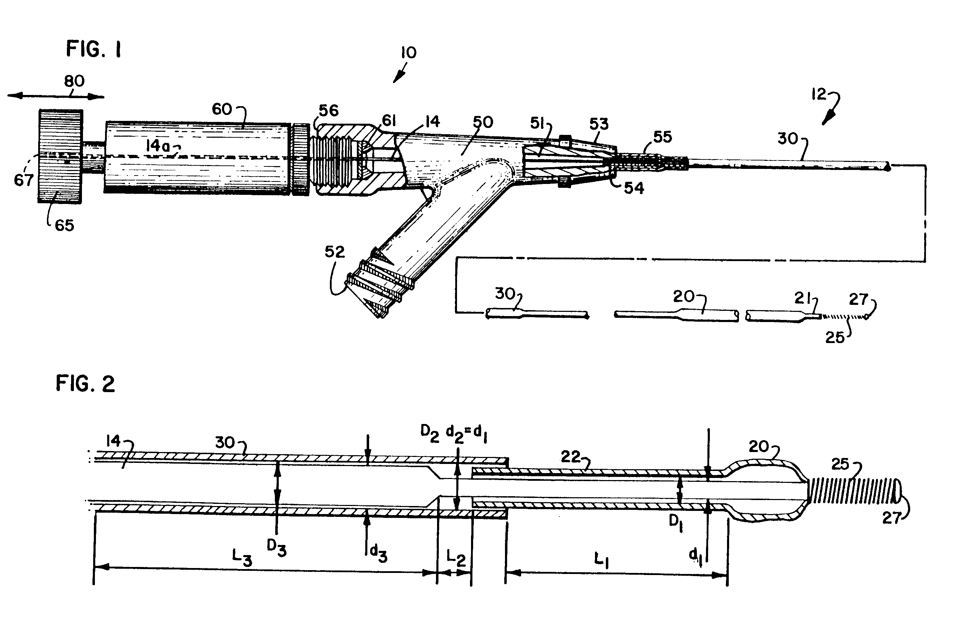

The construction of the preferred embodiment of the dilating guide wire is shown in Figures 1-5. Reference number 10 generally designates the control manifold assembly, while reference number 12 generally designates the dilating guide wire portion. Dilating guide wire portion 12 includes an inner, hollow core 14. Core 14 is made of stainless steel and extends the full length of the dilating guide wire and also substantially through the control manifold assembly 10. Core 14 is made of a suitable wall thickness and diameter for transmitting axial and torsional forces as the dilating guide wire is being advanced, while still maintaining an overall narrow diameter so that the dilating guide wire can fit through the guide wire-receiving lumen of a conventional angioplasty catheter. In the preferred embodiment the core has an outer diameter $d_3$ of 0.330 cm (.012 in.), which tapers or necks downwardly near the distal end to a diameter $d_1$ of 0.019 cm (.0075 in.) throughout the region where the balloon is. Beyond the balloon, core 14 tapers again as indicated at reference numbers 15 to a final narrow configuration. The tapered portion 15 can be formed by a compressional step that essentially closes off the interior hollow passage of core 14 at the tip.

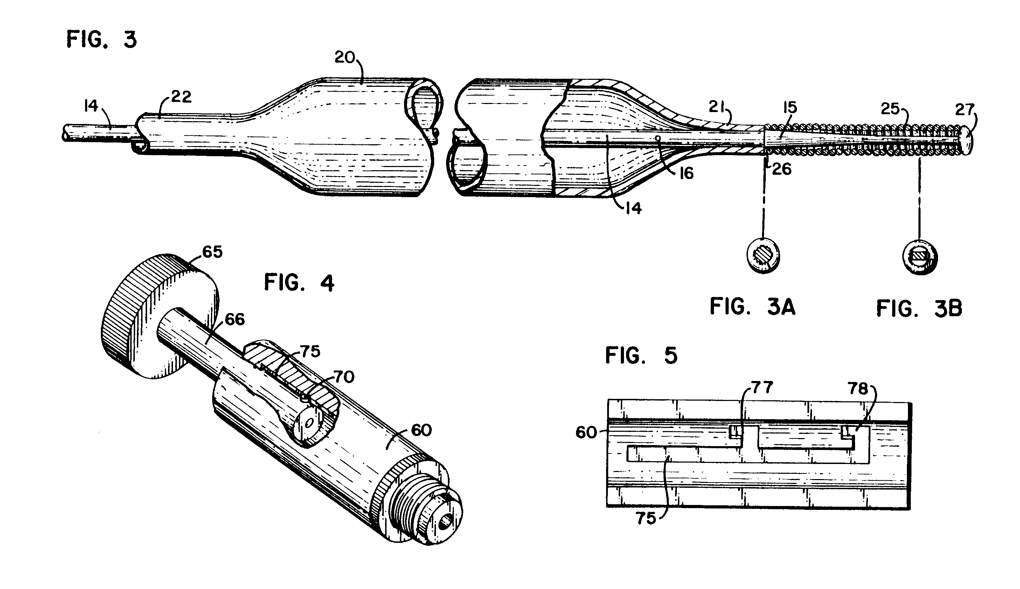

In the preferred embodiment, tapered portion 15 is tapered from a circular section, as indicated in Figure 3A, to a rectangular section as indicated at Figure 3B. Although circular, square or rectangular sections can he used, the rectangular section is used in the preferred embodiment because it has the advantage of greatest flexibility for bending due to the short dimension of the rectangular section, while still maintaining sufficient cross-sectional area to preserve ultimate yield strength. The greater flexibility of the rectangular section facilitates bending of the tip by the cardiologist prior to insertion for navigation purposes, and permits the tip to orient itself to various shapes within the artery.

Balloon 20 is positioned around core 14 near the distal end of the apparatus. Balloon 20 is formed from polyolefin. The distal end 21 of the balloon 20 is bonded to core 14. The proximal end 22 extends up to outer catheter tubing 30, and is secured thereto. In the preferred embodiment, the outside diameter of the dilating guide wire at the location of distal end 21 is .014 inch. The diameter in the balloon area when the balloon 20 is collapsed is 0.046 cm to 0.051 cm (.018 in. to .020 in.).

A hole 16 is provided in the core 14 approximately adjacent the distal end of the balloon to provide an air return path as is explained in greater detail below.

Outer catheter tubing 30 is made of polyethylene as is generally known in the art and is of somewhat greater outer diameter and stiffness than the balloon. Proximal end 22 of the balloon may fit inside the inner lumen of outer catheter tubing 30 for a short distance where it is bonded thereto. The proximal end of outer catheter tubing 30 extends up to and is joined to the control manifold assembly 10.

Referring again to Figure 3, the tip area includes a radiopaque flexible coiled spring 25 which is

4

positioned around tapered tip 15 of the hollow core. The diameter of the coiled spring tip area is 0.030 cm to 0.036 cm (.012 to .014 in.). Coil spring 25 is brazed to core 14 at the point indicated by reference number 26 where it abuts the distal end 21 of the balloon. A smoothly radiused safety button 27 is brazed to the distal end of coil spring 25 and hollow core 14. Gold brazing is the preferred form of brazing because it does not anneal the steel around the brazed area.

The control manifold assembly includes a main housing 50 which has an interior pressure manifold or chamber 51 formed therein. Hollow core 14 extends from the dilating catheter portion 12 all the way through housing 50 and its included pressure chamber 51. A branch of housing 50 forms the inflation port 52 which communicates internally with pressure chamber 51. Inflation port 52 may have a suitable threaded fitting as shown, and is used for connection to the apparatus for applying radiopaque fluid, which is generally known in the art, for causing controlled inflation of balloon 20.

Housing 50 has a distal end 53 which receives the dilating guide wire portion 12 and which forms the end of pressure chamber 51. A short piece of relatively stiff tubing 54 is molded into distal end 53 and extends a short distance outwardly therefrom. Tubing 54 is of sufficient diameter to allow hollow core 14 to extend therethrough with sufficient clearance to provide an annular lumen for inflation of the balloon. The outer catheter tubing 30 fits over tubing 54 and is bonded thereto. A strain relief 55, for example in the form of a piece of heat-shrink tubing, fits over outer catheter tubing 30 and tube 54 to help ensure mechanical security of the assembly. The annular lumen within the dilating guide wire between outer catheter tubing 30 and hollow core 14 communicates through tube 54 to pressure chamber 51.

At the other end of housing 50 there is formed a threaded bore 56 which receives a threaded tip of a control handle housing 60. Positioned in a recess of thread bore 56 is a pressure seal 61 in the form of an O-ring. This allows hollow core 14 to pass through from housing 50 into control handle housing 60, while sealing pressure chamber 51 and preventing passage of pressurized fluid past seal 61. A control knob 65 is attached to a shaft 66 which extends into a central bore in control handle housing 60, as also seen in Figure 4. Shaft 66 has a central bore 67 which receives hollow core 14. The proximal end of hollow core 14 is indicated by reference number 14a and is positioned within shaft 66 near the opening of central bore 67 at the end of the control knob 65. In this manner the interior core air passage of hollow core 14 is vented to the external atmosphere.

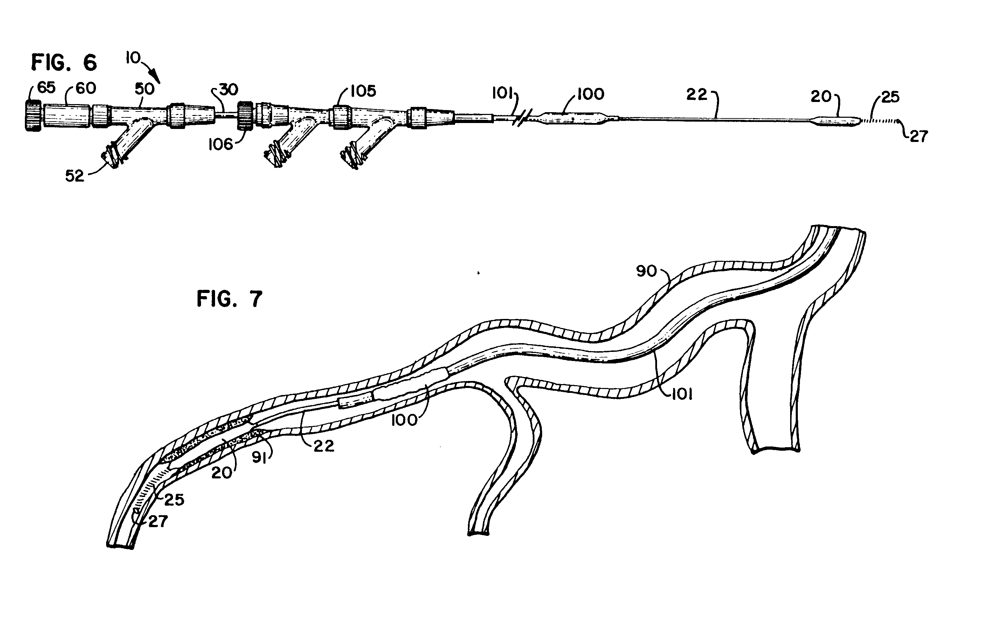

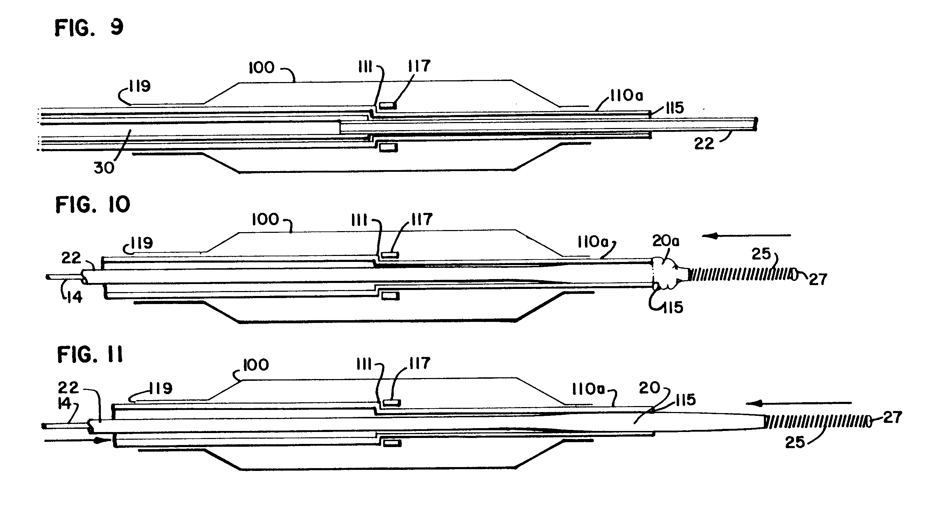

As seen in Figure 4, shaft 66 has a tab 70 projecting therefrom. This tab works in conjunction with grooves formed in the inner wall of central bore 62 of housing 60 to limit and define the permissible motion of shaft 66. As seen in Figures 4 and 5, the groove includes a longitudinally extending portion indicated by reference number 75. At the forward end of groove portion 75 it connects with a hook-shaped groove portion 78. At an intermediate position groove 75 connects with another hook-shaped groove portion 77. Knob 65 may move longitudinally of the apparatus as indicated by direction arrow 80 in Figure 1, through a limited range of motion determined by the length of groove 75.

Since the end of hollow core 14 is firmly secured in shaft 66 as by potting or the like, the above-described motion of control knob 65 causes corresponding axial movement of hollow core 14. Specifically, axial movement of knob 65 as indicated by direction arrow 80 causes axial movement of the core with respect to the outer catheter tubing 30 and causes axial stretching or relaxing of balloon 20. The system is designed so that when knob 65 is in the aft position, the balloon 20 is in the normal or unstressed condition. When knob 65 is pushed forward to bring tab 70 toward groove 78, this causes axial pushing of core 14 and stretching of balloon 20. Handle 65 can be rotated slightly to position tab 70 in the end of hook groove 78, which serves as a lock to hold the apparatus in the stretched position. The apparatus can similarly be locked with tab 70 in hook groove 77 to hold the apparatus in an intermediate position.

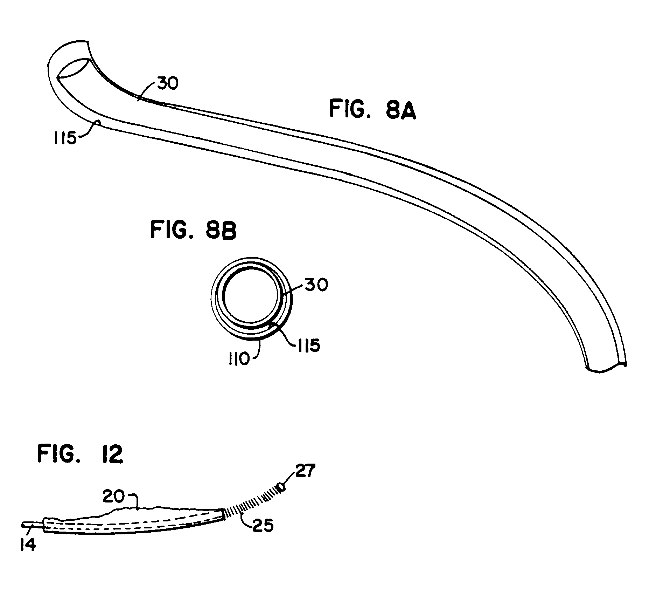

Axial stretching causes a reduction in profile or diameter of the balloon portion 20, as it is stretched out and thus positions itself more closely about core 14. This is explained in greater detail below with reference to Figures 10 and 11. When the tip area is not constrained inside a PTCA catheter, axial stretching can cause the tip portion including coil spring 25 to take a deflection toward one side, due to the nature of the axial force applied to core 14 and resisted by outer catheter tubing 30. This is indicated in Figure 12, where the side wall of balloon 20 opposite the deflection is pulled relatively smoothly along core 14. The other side of the balloon in the direction of the deflection is also stretched out somewhat to reduce the amount of slack, but is not pulled as tightly as the other side.

Use of the present invention is illustrated with reference to Figure 7, in which reference number 90 generally designates a portion of a patent's vascular system, specifically an artery and several branches thereof. Since the preferred embodiment of the invention is for use in treating the coronary arteries, the dilating guide wire is used in conjunction with a PTCA which is sized to cooperate with it. For that reason, the larger dilating catheter will be referred to herein as a PTCA catheter, but it will be understood that the dilating guide wire of the present invention may be used in conjunction with a dilating catheter for treatment of other arteries besides coronary arteries. The artery system includes a stenotic lesion 91 which is to be opened by angioplasty techniques. The first step is introducing a conventional guide wire in the customary manner and steering it into the appropriate branch of artery 90 to cross the lesion. (This step is not shown in Figure 6.) Next, a PTCA catheter is advanced over the conventional guidewire. If all goes well, it is advanced across the lesion so that its balloon can be inflated to open the stenosis. However, if the stenosis is very tight, the situation is encountered where the guide wire is successfully advanced across the lesion, but the PTCA catheter cannot cross the lesion because of its too-large diameter.

When this situation is encountered, the present invention can be advantageously employed in conjunction with a PTCA catheter of the type which has a central lumen, or through lumen, through which the dilating guide wire can pass. This means that the central lumen of the angioplasty catheter in the tip area thereof must accommodate the 0.051 cm (.020 in.) maximum outer diameter of the dilating guide wire, which is in the balloon area with the balloon collapsed, and also the slightly larger diameter of outer catheter tubing, which is not in the balloon or tip area, but which starts a distance therefrom and extends back to the manifold assembly. This is not a significant limitation on the PTCA catheter since many of them are designed to work with conventional guide wires of 0.030 cm to 0.046 cm (.012 to .018 in.). Therefore any of a number of conventional-type angioplasty catheters or PTCA catheters can be used, or a catheter especially designed to work with the dilating guide wire can be used, so long as it will receive the dilating guide wire.

In Figure 6, the dilating guide wire is inserted in the through lumen of the PTCA catheter. The PTCA catheter includes a balloon 100, a shaft portion 101, and a manifold assembly 105, which may be of conventional design. The through lumen of the catheter is accessible through fitting 106, and the dilating guide wire, distal tip first, has been threaded and extended through the through lumen so that the tip area including balloon 20 of the dilating guide wire extends beyond the balloon 100 of the PTCA catheter. In the preferred embodiment, the dilating guide wire balloon can extend up to 5.5 inches beyond the PTCA catheter balloon.

This condition is indicated also in Figure 9. The PTCA catheter includes an inner tubular member 110 which extends from the proximal to the distal end of the catheter. At reference number 111 in Figure 9, approximately midway within balloon 100, tubular member 110 steps to a smaller diameter, and at this reduced diameter extends out to the distal tip as indicated by reference number 110a. This reduction in diameter helps reduce the profile of the tip portion of the catheter and increases its flexibility. Tubular member 110 has a central lumen 115 which is the through lumen of the catheter. A radiopaque marker ring 117 is attached around member 110 at the approximate midpoint of balloon 100. The proximal end of the member forming balloon 100 connects to an outer tubular member (not shown) which extends up to the manifold assembly 105. An annular inflation lumen 119 is formed between this outer tubular member and inner tubular member 110.

Figure 9 shows the dilating guide ware inserted as far as it will go into the PTCA catheter. The step in outer diameter which occurs between the proximal end of the balloon material 22 and the distal end of outer catheter tubing 30 is accommodated by the step in inside diameter of tubular member 110 at 111. The balloon 20 of the dilating guide wire is not shown in Figure 9, but would be off the drawing to the right thereof, approximately 14.0 cm (5.5 in.) beyond balloon 100 of the PTCA catheter.

Referring again to the situation where the PTCA catheter has been advanced over the guide wire but cannot be advanced across the lesion, the PTCA catheter is held in place, and the guide wire is withdrawn and removed from the patient. The dilating guide wire according to this invention is then introduced through the central lumen of the PTCA catheter to replace the guide wire. When the tip of the dilating guide wire emerges from the tip of the PTCA catheter it is advanced further until it crosses the lesion, which is the position indicated in Figure 6. Balloon 20 of the dilating guide wire can then be inflated to partially compress lesion 91 and partially open the flow. The balloon and tip profile and the spring tip and core flexibility are very important in the ability to advance and cross the lesion. It is also important that the spring tip and inflated balloon are radiopaque so that the procedure can be monitored by fluoroscopy. After the balloon 20 has partly opened the flow in the artery, balloon 20 is then deflated and the dilating guide wire is advanced further down the coronary artery. The PTCA catheter is then advanced along the dilating guide wire to the lesion, which can now be successfully crossed since it has been partially dilated. The PTCA catheter can then be used in the usual manner to complete the angioplasty procedure and open the stenosis. If necessary, the conventional guide wire can be reinserted after removal of the dilating guide wire to aid in advancing the conventional catheter.

The present invention provides several useful features to aid in maneuvering the dilating guide wire to advance it across a lesion in an artery. First and foremost is the small effective diameter of the dilating guide wire, which permits it to be advanced through an angioplasty catheter in which has previously been maneuvered along a conventional guide wire to a short distance from the stenosis, typically 1.27 cm to 2.54 cm (.5 in. to 1.0 in.). Since the lesion may be around a turn in an artery or may be offset from the center of an artery, some maneuvering may still be needed. One possibility is pre-bending of the tip area as previously mentioned to give it a deflection which can then be steered by a rotation of the catheter. Another possibility is using the axial extension feature as previously discussed with reference to Figure 12, which also will deflect the tip area. A bent or deflected tip, viewed under fluoroscopy, can be steered to cross the lesion.

The steering is advantageously accomplished by rotating the entire angioplasty catheter-dilating guide wire assembly, which can be done by manipulating the control manifold assembly 105 for the PTCA catheter. There is sufficient contact between the outer surface of tubing 30 of the dilating guide wire and the inside of the through lumen of the angioplasty catheter so as to transmit torque from the latter to the former. In the preferred embodiment, the outer diameter of outer catheter tubing 30 is 0.066 cm (.026 in.), and the inner diameter of tubular member 110 of the PTCA catheter is 0.076 cm (.030 in.). This provides sufficient clearance to allow easy insertion and withdrawal of the dilating guide wire. However, once it is in place, there will be a waving, random distribution of surface contact zones between the catheters, as suggested in Figure 8A, as the two undergo a series of bends in various directions. This is also indicated in Figure 3B, which shows the catheters contacting along one edge, in response to a bend. Typically, blood and or contrast media occupy the space between the dilating guide wire and the inside wall of the through lumen of the PTCA catheter. These contact zones provide sufficient torque transmission that when the control manifold assembly 105 on the PTCA catheter is rotated, the PTCA catheter and the dilating guide wire rotate together as a unit. This takes advantage of the inherently greater torque transmission capability of the PTCA catheter, which it has by virtue of its thicker walls and larger diameter, and utilizes it for steering control of the relatively smaller and less stiff dilating guide wire. Also, the blood cells in and adjacent the zones of contact between the catheters tend to provide a high viscosity which aids in torque transmission. The net effect is that the cardiologist, viewing the procedure with the aid of fluoroscopy, has a very high degree of control over deflection of the dilating guide wire tip and steering of it to navigate the final inch or so to the lesion. The two can be advanced together as well as rotated.

Because of the small dimensions of the dilating guide wire and the demanding performance requirements it must meet, special fluid transport features and characteristics are provided. It is important that means are provided to completely purge the dilating guide wire shaft and balloon of air. This refers to the process of replacing all the air that occupied the volume of the catheter and balloon with a radiopaque contrast medium. This has been a problem with other types of catheters, and if some air remains in the balloon area it can change the radiopaque appearance of the inflated balloon, which causes difficulties for the cardiologist. The catheter must also be able to contain the pressure of the inflation applied to open the stenoses. Finally, deflation time for the balloon is very important, since the coronary artery is totally occluded during inflation of the balloon and the patient is in stress during that period. After the dilation is deemed complete by the cardiologist, it is very important that the balloon deflate rapidly, preferably in less than 20 seconds, so that blood flow is restored. The small dimensions of the balloon and core wire which are required in order to pass through the tip of a PTCA catheter can create difficulties in attaining rapid deflation rate. However, through consideration of the fluid transport parameters through the entire dilating guide wire catheter, an optimum design can be achieved which meets the low profile, strength and fluid transport requirements.

Figure 2 illustrates the important dimensions which determine the flow characteristics through the catheter. The predicted physical behavior of the flow rates can be attained using the following mathematical relation:

$Q = \Delta P/_{\epsilon Z}$ where $\Delta P$ = differential pressure driving the fluid

Q = fluid flow rate

Z = fluid-surface resistive factors, and

$$Z = \frac{8 \mu L}{\pi B R^4 C}$$

$\mu$ = fluid kinematic viscosity

L = length of fluid path

B = surface friction constant

R = inner radius of outer annulus channel

$$c = \left[ \left( 1 - \left(\frac{r}{R}\right)^4 \right) - \frac{\left( 1 - \left(\frac{r}{R}\right)^2 \right)^2}{\ln \left(\frac{R}{r}\right)} \right]$$

and r = outer radius of contained member of annulus. As can be soon from the above equation the relationship which includes a series of annuli which is the application in the fluid path of the dilating guide wire is a complex balance needed to provide the required profile that is needed to be contained within the inner lumenal path of the PTCA catheter while also providing a sufficient hydraulic channel for the balloon volume to deflate in the required 20-second maximum time frame.

In the preferred embodiment, the length $L_3$ of Figure 2 is 142.2 cm (56 in.); $L_2$ is 3.8 cm (1.5 in.); and $L_1$ is 14.0 cm (5.5 in.). Diameter $D_3 = D_2 = 0.051$ cm (.020 in.); diameter $d_3 = 0.030$ cm (.012 in.); $D_1 = 0.033$ cm (.013 in.); and, $d_1 = d_2 = 0.019$ cm (.0075 in.).

The purging of air from the catheter and balloon so that it can be completely filled with radiopaque contrast medium is taken care of by creating return path through the center of core 14 through which the air escapes when fluid is introduced. This path uses the central opening of hollow stainless steel drawn core 14 and aperture 16 provided therein near the distal end of the balloon. Aperture 16 is from 0.003 cm to 0.008 cm (.001 to .003 in.) in diameter and communicates between the outer fluid flow channel between the balloon and tubing 22 and core 14, and the hollow central lumen of core 14. During inflation air is forced through aperture 16 and out the core as the radiopaque fluid is introduced. After the balloon has been filled and the air removed, some of the radiopaque fluid will enter aperture 16 and proceed back up the central lumen of core 14 toward the proximal end thereof. The advance of this fluid is controlled by two factors. First, the fluid column is forced through the hole 16 by pressure applied to the fluid, and fluid flow is resisted to an extent by the small diameter of the hole. Second, once the fluid has entered the central lumen of core 16, capillary action which is governed by the surface tension between the fluid and the central lumen surface characteristics and diameter will allow the fluid into the core along a certain length until a state of equilibrium is reached. At this point, more pressure would be required to begin movement of the fluid in the column than the catheter can be subject to, and therefore the fluid advances no further. This applies to both the application of positive pressure and vacuum. For this reason, the catheter is not only ventable through aperture 16 and the hollow core, but is also self-sealing. No additional seal or valve is required to prevent fluid and pressure from bleeding off through this vent passage. The flow characteristics of the radiopaque fluid in the core are dependent on optimization of the capillary action and static breakaway pressure shears.

It will be appreciated that due to the small diameter of spring 25 and the low profile of balloon 20 in its deflated state, the dilating guide wire can be advanced through a tight stenosis essentially anywhere that can be crossed by a conventional guide wire. The balloon 20 when slack tends to streamline itself and lie back along core 14 as the device is being advanced, thus providing a low profile. This low profile can be enhanced even further by pushing knob 65 forward, as discussed above, to axially stretch balloon 20 and thus further reduce its profile.

The axial stretching of the balloon is very important in withdrawing the balloon of the dilating guide wire into the PTCA catheter to remove it. After the inflation of balloon 20 has been completed, pressure is removed and the balloon deflated. At this point, if one were to attempt to withdraw the dilating guide wire back into the conventional catheter without the axial stretch feature of this invention, the undesirable effect illustrated in Figure 10 would take place. The proximal end of the balloon 20 would withdraw into the PTCA catheter as the deflated balloon 20 would tend to streamline along hollow core 14. However, the excessive slack of the balloon, now larger due to its having been stretched in the dilating process, would tend to bunch up or accordion toward the distal end as indicated at reference number 20a, and this bunching would prevent the dilating guide wire from being fully withdrawn into and through the central lumen 115 of the PTCA catheter. To solve this problem, the axial stretch feature of the present invention is used to stretch the balloon, thus drawing it thinner and lowering its profile to permit complete withdrawal through lumen 115 as illustrated in Figure 11. In Figure 11, the control knob 65 has been pushed forward and rotated to lock tab 70 in hook-shaped groove 78. This pushes the core 14 toward the distal end and stretches the balloon. This might be needed to reinsert the guide wire to advance further along the artery, and without the axial stretch feature to permit withdrawal of the dilating guide wire, it would be necessary to remove the PTCA catheter also, which would result in loss of the path.

The present invention thus provides an extremely low profile dilating guide wire for predilation of tight stenoses which cannot be crossed by a conventional PTCA or other angioplasty catheter. The low profile permits the device to be inserted through the PTCA in place of a guide wire, without loss of the path to the stenosis. The dilating guide wire together with a PTCA or other angioplasty catheter sized to receive it form a system which can be easily steered and manipulated for predilation and subsequent dilation of the stenosis more effectively than previously feasible with other catheter types.

**Claims**

1. Angioplasty apparatus for use in opening of very tight stenoses in the coronary arteries, said apparatus including a percutaneous transluminal coronary angioplasty (PTCA) catheter with an elongate first tubular member (101) having a through lumen and an inflatable first balloon (100) adjacent a distal end thereof, small enough with the first balloon uninflated to fit in a coronary artery, and including first control means (105) at the proximal end thereof for controlling motion of the PTCA catheter in the vascular system and inflation of the first balloon in an angioplasty procedure, said apparatus being characterized by a second catheter, adapted for fitting in the through lumen of said PTCA catheter, including an elongate, relatively stiff core (14) and an elongate, relatively flexible hollow second tubular member (30) disposed generally coaxially around and along said core, said apparatus also having an inflatable second balloon (20) located at the distal end of the second tubular member of said second catheter with its distal end (21) sealed to the distal end of said core, and second control means (60, 65) attached to the proximal end of said second catheter to control motion thereof and inflation of said second balloon with inflation fluid through an annular passage between said second tubular member and said core, said second catheter having means (16, 67) for venting said second balloon, said second catheter having an outside diameter small enough to permit introduction into the through lumen of said PTCA catheter through an access in the first control means thereof, and movement through said lumen to extend the second balloon of said second catheter beyond the first balloon and distal tip of said PTCA catheter, whereby said second catheter may be used to predilate a stenosis to open it sufficiently for subsequent crossing and dilation by said PTCA catheter without requiring the loss of and reestablishment of the path across the stenosis.

2. Apparatus according to claim 1 wherein said second control means (60, 65) includes means (51, 52), attached to the proximal end of said second tubular member (30), for fluid communication with said annular passage for inflation and deflation of said second balloon.

3. Apparatus according to claim 1 wherein said second control means (60, 65) includes means (65) attached to said core member (14) for selectively applying axial force thereto relative to holding said second tubular member (30) to effect axial stretching and reduction of profile of said second balloon (20).

4. Apparatus according to claim 3 wherein the outside diameter of the distal end of said second catheter with the second balloon (20) deflated is about .02 inch or less.

5. Apparatus according to claim 1 wherein said venting means (16, 67) includes a fluid vent passage (57) in said core from its proximal end to a vent opening (16) in a portion thereof within said second balloon (20) to provide a vent path for removing air from said second balloon as it is inflated with pressurized inflation fluid.

6. Apparatus according to claim 5 wherein said vent passage (67) has small enough internal dimensions to cause self sealing of inflation fluid entering therein as said second balloon (20) is inflated duo to capillary action and surface tension of the inflation fluid in the vent passage.

7. Apparatus according to claim 1 further including a flexible spring safety tip (25) attached to said core (14) at the distal end thereof.

8. Apparatus according to claim 7 wherein said means for applying axial force includes means for locking said core with respect to said second tubular member with said second balloon in the stretched condition.

9. Apparatus according to claim 1 wherein said core extends beyond the point of attachment of the distal end of said balloon and tapers to a smaller diameter non-hollow region and further including a flexible safety spring disposed about and attached to the tip of said core.

10. Apparatus according to claim 9 wherein said tip portion of said core tapers to a thin rectangular sectioned ribbon, to provide greater flexibility.

11. Apparatus according to claim 1 wherein said second catheter fits within said PTCA catheter with sufficient contact for torque transmission so that the distal end of said second catheter extending beyond said PTCA catheter can be rotated for steering by manual rotation of the system consisting of said PTCA catheter and said second catheter.

12. Apparatus according to claim 1 wherein said second balloon (20) of said second catheter has a diameter greater than the uninflated diameter of the first balloon (100) of the PTCA catheter, so that the second catheter can be used to predilate a stenosis to open it sufficiently for subsequent crossing and dilation by said PTCA catheter without requiring the loss of and reestablishing of the path across the stenosis.

**Patentansprüche**

1. Angioplastiegerät zur Verwendung beim Öffnen sehr enger Stenosen in den Koronararterien, wobei das Gerät einen perkutanen transluminalen Koronarangioplastie (PTCA) - Katheter mit einem länglichen ersten röhrenförmigen Bauteil (101), das einen durchgängigen Hohlraum und einen aufblasbaren ersten Ballon (100) nahe seinem distalen Ende besitzt, klein genug, um bei nicht aufgeblasenem ersten Ballon in eine Koronararterie zu passen, umfaßt und eine erste Steuereinrichtung (105) an seinem proximalen Ende, um die Bewegung des PTCA-Katheters im vaskularen System und das Aufblasen des ersten Ballons bei einem Angioplastieverfahren zu steuern, umfaßt, wobei das Gerät **gekennzeichnet** ist durch einen zweiten Katheter, so ausgelegt, daß er in den durchgängigen Hohlraum des PTCA-Katheters paßt, mit einem länglichen, vergleichsweise steifen Kern (14) und einem länglichen, relativ flexiblen, hohlen zweiten röhrenförmigen Bauteil (30), das im allgemeinen koaxial um und entlang des Kerns angeordnet ist, wobei das Gerät zudem einen aufblasbaren zweiten Ballon (20), am distalen Ende des zweiten röhrenförmigen Bauteils des zweiten Katheters angeordnet, aufweist, dessen distales Ende (21) mit dem distalen Ende des Kerns abgedichtet verbunden ist, und durch eine zweite Steuereinrichtung (60, 65), die mit dem proximalen Ende des zweiten Katheters verbunden ist, um dessen Bewegung und das Aufblasen des zweiten Ballons mittels eines Füllfluids durch einen ringförmigen Durchlaß zwischen dem zweiten röhrenförmigen Bauteil und dem Kern zu steuern, wobei der zweite Katheter ein Mittel (16, 67) zum Entlüften des zweiten Ballons besitzt, wobei der zweite Katheter einen Außendurchmesser hat, der klein genug ist, um das Einführen in den durchgängigen Hohlraum des PTCA-Katheters durch einen Zugang in seiner ersten Steuereinrichtung und eine Bewegung durch den Hohlraum zu erlauben, so daß der zweite Ballon des zweiten Katheters über den ersten Ballon und das distale Ende des PTCA-Katheters hinaus ausgedehnt wird, wodurch der zweite Katheter zum Vorweiten einer Stenose benutzt werden kann, um diese hinreichend für das nachfolgende Durchführen und Aufweiten durch den PTCA-Katheter zu öffnen, ohne daß der Weg durch die Stenose verlorengeht und wiederhergestellt werden muß.

2. Gerät nach Anspruch 1, bei dem die zweite Steuereinrichtung (60, 65) eine mit dem proximalen Ende des zweiten röhrenförmigen Bauteils (30) verbundene Einrichtung (51, 52) für die Fluidverbindung mit dem ringförmigen Durchlaß zum Aufblasen und Entleeren des zweiten Ballons umfaßt.

3. Gerät nach Anspruch 1, bei dem die zweite Steuereinrichtung (60, 65) eine Einrichtung (65) enthält, die mit dem Kernstück (14) verbunden ist und mit der selektiv darauf eine axiale Kraft in bezug auf das Festhalten des zweiten röhrenförmigen Bauteils (30) ausgeübt werden kann, um bei dem zweiten Ballon (20) eine axiale Dehnung und eine Reduktion des Querschnitts zu bewirken.

4. Gerät nach Anspruch 3, bei dem der Außendurchmesser des distalen Endes des zweiten Katheters für den Fall, daß der zweite Ballon (20) nicht aufgeblasen ist, 0,02 Zoll oder kleiner ist.

5. Gerät nach Anspruch 1, bei dem das Mittel zum Entlüften (16, 67) einen Fluidentlüftungsweg (67) in dem Kern von dessen proximalem Ende zu einer Entlüftungsöffnung (16) in einem seiner Bereiche innerhalb des zweiten Ballons (20) enthält, um einen Entlüftungsweg zum Entfernen der Luft aus dem zweiten Ballon zu ermöglichen, wenn dieser mittels eines unter Druck stehenden Füllfluids aufgeblasen ist.

6. Gerät nach Anspruch 5, bei dem die inneren Abmessungen des Entlüftungswegs (67) klein genug sind, um eine Selbstabdichtung des darin eintretenden Füllfluids aufgrund der Kapillarwirkung und der Oberflächenspannung des Füllfluids in dem Entlüftungsweg zu bewirken, wenn der zweite Ballon (20) aufgeblasen ist.

7. Gerät nach Anspruch 1, das weiterhin eine elastische Federsicherheitsspitze (25) besitzt, die mit dem Kern (14) an dessen distalem Ende verbunden ist.

8. Gerät nach Anspruch 7, bei dem die Einrichtung zum Ausüben einer axialen Kraft eine Einrichtung enthält, mit der der Kern relativ zu dem zweiten röhrenförmigen Bauteil arretiert werden kann, wenn der zweite Ballon ausgedehnt ist.

9. Gerät nach Anspruch 1, bei dem der Kern sich über den Befestigungspunkt des distalen Endes des Ballons erstreckt und sich zu einem nicht hohlen Bereich mit kleinerem Durchmesser verjüngt, und das weiterhin eine elastische Sicherheitsfeder enthält, die um die Spitze des Kerns herum angeordnet und an dieser angebracht ist.

10. Gerät nach Anspruch 9, bei dem der Spitzenbereich des Kerns sich zu einem dünnen Band mit rechteckigem Querschnitt verjüngt, um größere Flexibilität zu ermöglichen.

11. Gerät nach Anspruch 1, bei dem der zweite Katheter mit hinreichendem Kontakt für die Übertragung eines Drehmoments in den PTCA-Katheter paßt, so daß das distale Ende des zweiten Katheters, das sich über den PTCA-Katheter hinaus erstreckt, zur Steuerung durch manuelle Rotation des aus dem PTCA-Katheter und dem zweiten Katheter bestehenden Systems gedreht werden kann.

12. Gerät nach Anspruch 1, bei dem der zweite Ballon (20) des zweiten Katheters einen größeren Durchmesser als den des nicht aufgeblasenen ersten Ballons (100) des PTCA-Katheters besitzt, so daß der zweite Katheter zum Vorweiten einer Stenose benutzt werden kann, um diese hinreichend für das nachfolgende Durchführen und Aufweiten durch den PTCA-Katheter zu öffnen, ohne daß der Weg durch die Stenose verloren geht und wiederhergestellt werden muß.

**Revendications**

1. Appareil d'angioplastie utilisé pour ouvrir les sténoses très serrées des artères coronaires, ledit appareil comprenant un cathéter percutané transluminal d'angioplastie coronarienne (PTAC) incluant un premier élément tubulaire allongé (101) pourvu d'une lumière traversante et d'un premier ballon gonflable (100) adjacent à l'extrémité distale de celui-ci, suffisamment petit avec le premier ballon dégonflé pour être installé dans une artère coronaire, et incluant des premiers moyens de contrôle (105) à l'extrémité proximale de celui-ci pour contrôler le mouvement du cathéter PTAC dans le système vasculaire et pour contrôler le gonflage du premier ballon au cours de l'opération d'angioplastie, ledit appareil étant caractérisé en ce qu'il comprend un second cathéter, apte à être installé dans la lumière traversante dudit cathéter PTAC, incluant une partie centrale (14) allongée relativement rigide et un second élément tubulaire (30) creux relativement flexible disposé essentiellement coaxialement autour et le long de ladite partie centrale, ledit appareil présentant également un second ballon gonflable (20) situé à l'extrémité distale du second élément tubulaire dudit second cathéter et dont l'extrémité distale (21) est scellée à l'extrémité distale de ladite partie centrale, et des seconds moyens de contrôle (60, 65) fixés à l'extrémité proximale dudit second cathéter pour contrôler le mouvement de celui-ci et le gonflage dudit second ballon avec un fluide de gonflage passant par un passage annulaire entre ledit second élément tubulaire et ladite partie centrale, ledit second cathéter présentant des moyens de purge (16, 67) dudit second ballon, ledit second cathéter présentant un diamètre extérieur suffisamment petit pour permettre son introduction dans la lumière traversante dudit cathéter PTAC par un accès situé dans les premiers moyens de contrôle de celui-ci, et pour permettre un mouvement dans ladite lumière de façon à étendre le second ballon dudit second cathéter au-delà du premier ballon et au-delà de l'extrémité distale dudit cathéter PTAC, ledit second cathéter pouvant être utilisé pour prédilater une sténose de façon à l'ouvrir suffisamment afin qu'elle puisse ensuite être traversée et dilatée par ledit cathéter PTAC sans risque de perdre et d'avoir ensuite à rétablir le chemin à travers cette sténose.

2. Appareil selon la revendication 1 dans lequel lesdits seconds moyens de contrôle (60, 65) incluent des moyens (51, 52) fixés à l'extrémité proximale dudit second élément tubulaire (30), permettant la communication de fluide avec ledit passage annulaire lors du gonflage et du dégonflage dudit second ballon.

3. Appareil selon la revendication 1 dans lequel lesdits seconds moyens de contrôle (60, 65) incluent des moyens (65) fixés à ladite partie centrale (14) permettant d'appliquer sélectivement sur celle-ci une force axiale relative au maintient duddit second élément tubulaire (30) pour effectuer un étirement axial et une diminution du profil dudit second ballon (20).

4. Appareil selon la revendication 3 dans lequel le diamètre extérieur de l'extrémité distale dudit second cathéter avec le second ballon (20) dégonflé est inférieur ou égal à environ 0,02 pouce.

5. Appareil selon la revendication 1 dans lequel lesdits moyens de purge (16, 67) incluent un passage d'évacuation de fluide (67) situé dans ladite partie centrale et allant de son extrémité proximale jusqu'à un évent (16) situé dans une partie de celle-ci à l'intérieur dudit second ballon (20) constituant un chemin d'évacuation pour enlever l'air du second ballon lorsqu'il est gonflé avec un fluide de gonflage pressurisé.

6. Appareil selon la revendication 5 dans lequel ledit passage d'évacuation (67) présente des dimensions internes suffisamment petites pour être auto-étanche au fluide de gonflage y entrant lorsque ledit second ballon (20) est gonflé, du fait de l'action capillaire et de la tension de surface du fluide de gonflage dans le passage d'évacuation.

7. Appareil selon la revendication 1 incluant de plus une extrémité de sûreté flexible à ressort (23) fixée à ladite partie centrale (14) au niveau de l'extrémité distale de celle-ci.

8. Appareil selon la revendication 7 dans lequel lesdits moyens pour appliquer une force axiale incluent des moyens pour bloquer ladite partie centrale par rapport audit second élément tubulaire avec ledit second ballon en position étirée.

9. Appareil selon la revendication 1 dans lequel ladite partie centrale s'étend au-delà du point de fixation de l'extrémité distale dudit ballon et présente un diamètre s'amincissant jusqu'à une région non creuse, et inclut de plus un ressort de sécurité flexible disposé autour de et fixé à l'extrémité de ladite partie centrale.

10. Appareil selon la revendication 9 dans lequel ladite partie d'extrémité de ladite partie centrale s'amincit en un ruban fin de section rectangulaire, procurant une plus grande flexibilité.

11. Appareil selon la revendication 1 dans lequel ledit second cathéter s'adapte audit cathéter PTAC selon un contact suffisant pour permettre une transmission de torsion de sorte que l'extrémité distale dudit second cathéter s étendant au-delà dudit cathéter PTAC puisse être mise en rotation pour permettre un guidage par rotation manuelle du système constitué par ledit cathéter PTAC et par ledit second cathéter.

12. Appareil selon la revendication 1 dans lequel ledit second ballon (20) dudit second cathéter présente un diamètre supérieur au diamètre dégonflé du premier ballon (100) du cathéter PTAC, de telle sorte que le second cathéter puisse être utilisé pour prédilater une sténose afin de l'ouvrir suffisamment pour qu'elle puisse ensuite être traversée et dilatée par ledit cathéter PTAC sans risque de perdre et d'avoir ensuite à rétablir le chemin à travers cette sténose.

FIG. 1

FIG. 2

FIG. 3

FIG. 3A

FIG. 3B

FIG. 4

FIG. 5

FIG. 6

FIG. 7

EP 0 280 728 B1

FIG. 9

FIG. 10

FIG. 11

EP 0 280 728 B1

FIG. 8A

30

115

FIG. 8B

30

115

110

FIG. 12

20

27

14

25